# NEUE EUROPÄISCHE PATENTSCHRIFT

(11) **EP 1 105 002 B2**
(45) Veröffentlichungstag und Bekanntmachung des Hinweises auf die Entscheidung über den Einspruch: **03.10.2012**
(45) Hinweis auf die Patenterteilung: 17.07.2002
(21) Anmeldenummer: 99941588.8
(22) Anmeldetag: 11.08.1999
(51) Int. Cl.: A23L 1/09, A23L 1/308

(54) **KOHLENHYDRATMISCHUNGEN**
CARBOHYDRATES MIXTURE
MELANGES D'HYDRATES DE CARBONE

(30) Priorität: 11.08.1998 DE 19836339
(43) Veröffentlichungstag der Anmeldung: 13.06.2001
(73) Patentinhaber: N.V. Nutricia, 2712 HM Zoetermeer (NL)
(72) Erfinder: SAWATZKI, Günther, D-35516 Münzenberg (DE); STAHL, Bernd, D-61381 Friedrichsdorf (DE)
(74) Vertreter: Jorritsma, Ruurd
(86) Internationale Anmeldenummer: PCT/EP1999/005878
(87) Internationale Veröffentlichungsnummer: WO 2000/008948

(56) Entgegenhaltungen:
- EP-A- 0 504 055
- EP-A- 0 756 828
- WO-A-96/13271
- WO-A-98/26787
- US-A- 5 776 887
- I. MASAKI ET AL.: 'Influence of galactooligosaccharides on the human fecal microflora' J. NUTR. SCI. VITAMINOL Bd. 39, 1993 - 1993, JAPAN, Seiten 635 - 640
- RAFTIMIX 10, PRODUCT SHEET Nr. 05/95,

## Beschreibung

Die Erfindung betrifft Kohlenhydratmischungen für diätetische Nahrungen und Pharmazeutika, diese Kohlenhydratmischungen enthaltende diätetische und pharmazeutische Mittel sowie die Verwendung dieser Kohlenhydratmischungen zur Förderung der humanen Dickdarmflora.

Kohlenhydrate stellen bekanntlich einen der wesentlichen Grundpfeiler der Ernährung dar. Daher werden die unterschiedlichsten Kohlenhydrate den verschiedensten Nahrungen und auch Pharmazeutika beigegeben. Die Aufgabe der Kohlenhydrate ist daher primär nutritiver Art bzw. sie fungieren als Ballaststoff.

Die Kohlenhydrate bestehen aus Monosacchariden bzw. setzen sich aus diesen zusammen. Je nach Polymerisationsgrad werden die Kohlenhydrate als Oligosaccharide bzw. Polysaccharide oder Glycane bezeichnet. Die Kohlenhydrate liegen dabei sowohl als freie Oligosaccharide als auch in gebundener Form vor, beispielsweise in Glycoproteinen, Proteoglycanen und Glycolipiden.

Aufgrund der Variabilität der die Kohlenhydrate aufbauenden Monomere, der Position der glycosidischen Bindung und der Anomerie der Kohlenhydrate und deren Konjugate stellen diese Kohlenhydrate und deren Konjugate eine extrem heterogene und umfangreiche Substanzklasse dar.

Kohlenhydrate haben nun die unterschiedlichsten biologischen Funktionen. So beeinflussen sie beispielsweise die bakterielle Besiedlung des Dickdarmes, die eine Voraussetzung für dessen normale Funktion ist. Die Mikroflora des Dickdarmes greift auf sehr komplexe Weise in die intestinalen Funktionen ein. Dieser Einfluß wird vor allem durch die Fermentlerung von im Dünndarm nicht resorbierten Nahrungsbestandteilen ausgeübt. Die Fermentierung schließt eine Vielzahl von Funktionen wie den weiteren Aufschluß dieser Nahrungsbestandteile, die Entgiftung von endogenen entstandenen Metaboliten, die Synthese von neuen Metaboliten mit zum Teil sehr spezifischer Wirkung, die Rückresorption von Gallensäuren und viele andere Prozesse ein. Die normale Mikroflora wirkt auch dadurch gesundheitsfördernd, daß sie das Wachstum anderer pathogener Mikroorganismen unterdrückt.

Bakterien, die Milchsäure als ihr wichtigstes Stoffwechselendprodukt erzeugen (sog. Milchsäurebakterien) spielen als wichtige Vertreter der normalen Mikroflora des Dickdarmes eine ganz wesentliche Rolle. Beispiele für die Gruppe sind Bakterien der Genera Lactobacillus und Bifidobacterium. Es werden darum schon seit längerer Zeit Bemühungen unternommen, durch diätetische Maßnahmen die Entwicklung einer milchsäurebakterien-dominanten Darmflora zu steuern. Dies ist besonders dann wichtig, wenn entweder durch entwicklungsbedingte Prozesse wie z.B. bei Neugeborenen oder durch krankhafte Zustände wie z.B. nach enteraler antibiotischer oder anderer medikamentöser Therapie oder während oder nach enteralen Infektionen eine normale Darmflora nicht oder nicht ausreichend vorhanden ist.

Kohlenhydrate werden nun zunehmend in Nahrungen, "Functional Food" und Pharmazeutika unter dem Aspekt einer biologischen Wirksamkeit eingesetzt. So ist es beispielsweise bekannt, daß einige Kohlenhydrate einen wachstumsfördernden Effekt auf verschiedene Spezies der Bifidobakterien als auch der Lactobacilli aufweisen. So haben beispielsweise Galacto-Oligosaccharide einen wachstumsfördernden Effekt auf Lactobacillus casei. Bisher wurden jedoch nur sehr spezielle, über eine bestimmte Eigenschaft verfügende Kohlenhydratspezies zur Förderung bestimmter biologischer Wirkungen eingesetzt.

So beschreibt beispielsweise die WO 98/26787 den Einsatz von beta-Glucan und von davon abgeleiteten Verbindungen zur Förderung der Population von Milchsäure produzierenden Mikroorganismen im Gastrointestinaltrakt von Mensch und Tier. Auch können Mischungen Anwendung finden, die weitere präbiotische Substanzen enthalten, wobei letztere jedoch nicht genauer spezifiziert sind.

Ferner sind aus der WO 96/13271 Mischungen bekannt, die neben Immunglobulinen auch verschiedene Oligo- und und Polysaccharide enthalten. Diese Mischungen werden als diätetisches Supplement eingesetzt, das bei oraler Verabreichung gegen verschiedene gastrointestinale Pathogene wirksam sein soll. Die eingesetzten Saccharide werden dabei als lösliche diätetische Faser bezeichnet, wobei es sich um Inulin, Fructo-Oligosaccharide, Pectin, Guargummi und Mischungen davon handelt.

Aus der EP 0 756 828 A1 sind ferner faserhaltige Nährmittelzusammensetzungen beschrieben, die neben Oligosacchariden und/oder Stärke lösliche, keine Stärke darstellenden Polysaccharide und unlösliche, keine Stärke darstellenden Polysaccharide enthalten.

Aufgabe der vorliegenden Erfindung ist es, verbesserte Kohlenhydratmischungen bereitzustellen, die diätetischen Nahrungen sowie Pharmazeutika einverleibt werden können und neben einem nutritiven Effekt auch gesundheitsfördernde Mikroorganismen, die in der natürlichen Dickdarmflora vorhanden sind, stimulieren.

Gelöst wird diese Aufgabe durch Kohlenhydratmischungen gemäß der Lehre der Ansprüche.

Die erfindungsgemäßen Kohlenhydratmischungen enthalten somit mindestens zwei unterschiedliche, im wesentlichen lösliche Kohlenhydratkomponenten A und B, die im Magen-Darm-Trakt unverdaut bleiben und nicht resorbiert bis zum Dickdarm gelangen. Die erfindungsgemäßen Kohlenhydratmischungen können auch ausschließlich aus diesen beiden Kohlenhydratkomponenten A und B bestehen.

Die Kohlenhydratkomponente A besteht dabei mindestens aus einem Monosaccharid oder aus mindestens einem Oligosaccharid. Als Oligosaccharide werden dabei solche mit 2 bis 6 Monosaccharideinheiten verstanden. Bei den Oligosacchariden handelt es sich somit um Di-, Tri-, Tetra-, Penta- und Hexasaccharide. Die Kohlenhydratkomponente A kann auch aus einer Mischung aus zweien oder mehreren der genannten Saccharide aufgebaut sein. Sie kann somit nur aus einem Monosaccharid oder aus einer Mischung von zweien oder mehreren Monosacchariden oder aus einer Mischung aus einem Monosaccharid oder mehreren Monosacchariden mit einem Oligosaccharid oder mehreren Oligosacchariden bestehen. Sie kann auch aus einer beliebig großen Anzahl verschiedener derartiger Monosaccharide und/oder Oligosaccharide bestehen.

Die Kohlenhydratkomponente B besteht aus mindestens einem Polysaccharid mit 7 oder mehr Monosaccharideinheiten. Als Polysaccharide werden dabei solche ab Heptasaccharid (beispielsweise Hepta-, Okta-, Nona-, Decasaccharid usw.) verstanden. Auch die Kohlenhydratkomponente B kann aus nur einem derartigen Polysaccharid oder aus einer beliebig großen Anzahl von derartigen Polysacchariden bestehen.

Wenn daher nachstehend und auch in den Patentansprüchen von einer Kohlenhydratkomponente A bzw. B die Rede ist, dann kann es sich um alle diese verschiedenen Varianten handeln.

Die Kohlenhydratkomponente A macht dabei 90 Gew.-% der Summe der Kohlenhydratkomponente A und der Kohlenhydratkomponente B (A+B=100 Gew.-%) aus. Die Kohlenhydratkomponente B macht 10 Gew.-% der Summe aus der Kohlenhydratkomponente A und der Kohlenhydratkomponente B aus.

Mindestens 80 Gew.-% der Kohlenhydrate bzw. Saccharide der Summe der Kohlenhydratkomponente A und B wirken dabei präbiotisch. Vorzugsweise wirken mindestens 80 Gew.-% der zur Kohlenhydratkomponente A gehörigen Kohlenhydrate und auch mindestens 80 Gew.-% der zur Kohlenhydratkomponente B gehörenden päbiotisch. Anders ausgedrückt, vorzugsweise mindestens jeweils 80 Gew.-% der Kohlenhydrate bzw. der Saccharide der Kohlenhydratkomponenten A und B müssen unverdaut (und daher nicht im Dünndarm resorbierbar) in den Dickdarm gelangen. Mit anderen Worten, diese Kohlenhydrate bzw. Saccharide der Kohlenhydratkomponenten A und B werden im Magen-Darm-Trakt weder im Magen noch im Dünndarm resorbiert und verdaut, sondern gelangen als solche in den Dickdarm.

Der Anteil der nicht präbiotisch wirkenden Kohlenhydrate bzw. Saccharide bei den Kohlenhydratkomponenten A und B beträgt somit maximal 20 Gew.-%. Bei diesen Kohlenhydraten bzw. Sacchariden handelt es sich um solche, die zwar löslich sind, jedoch unverdaut ausgeschieden werden können. Diese Kohlenhydrate können einen physikalischen Effekt bewirken, indem Sie beispielsweise das Stuhlvolumen erhöhen oder aber eine Wasserbindung ausüben.

Als lösliche Kohlenhydrate im Sinne der Erfindung sind solche zu verstehen, die in Wasser in einer Konzentration von mindestens 1g/l bei Raumtemperatur eine homogene Lösung im physikalischen Sinne (z.B. gemäß Römpps Chemie Lexikon) bilden.

Wie bereits dargelegt, können die erfindungsgemäßen Kohlenhydratmischungen ausschließlich aus den Kohlenhydratkomponenten A und B bestehen oder diese enthalten. Zur Bestimmung des Anteiles, welche die Kohlenhydratkomponenten A und B beispielsweise in einem diätetischen oder pharmazeutischen Produkt ausmachen, geht man wie folgt vor:

In einer ersten Stufe werden alle löslichen Kohlenhydrate aus dem Produkt mit Wasser extrahiert. Fette und Proteine werden aus dem Extrakt entfernt.

In einer zweiten Stufe werden die löslichen Kohlenhydrate bzw. der Extrakt mit humanen Enzymen, beispielsweise humaner Amylase, humanem Pankreassekret oder Dünndarm-Bürstensaumpräparation, verdaut. Die dabei resultierenden nicht-verdauten Kohlenhydrate (mit Ausnahme der in diesem in-vitro Experiment entstehenden, in-vivo-resorbierbaren Monosaccharide) machen die beiden Kohlenhydratkomponenten A und B aus und müssen zu 80 % präbiotisch wirken.

Unter einem präbiotisch wirkenden Kohlenhydrat wird erfindungsgemäß ein solches verstanden, das unverdaut (und daher nicht im Dünndarm resorbierbar) in den Dickdarm gelangt und dort selektiv das Wachstum und/oder die Aktivität von einer oder einer begrenzten Zahl bakterieller Spezies im Darm begünstigt und daher die Gesundheit fördert. Diese präbiotische Wirkung derartiger Kohlenhydrate und deren genauere Wirkungsweise sind näher beschrieben in "G. R. Gibson & M. B. Roberfroid, J. Nutr. 1995; 125: 1401 - 1412", worauf hiermit ausdrücklich Bezug genommen und zum Offenbarungsgehalt der vorliegenden Unterlagen gemacht wird.

Erfindungsgemäße Kohlenhydratmischungen sind somit solche, bei denen die im oben beschriebenen Sinne löslichen und unverdauten Kohlenhydrate die hier näher beschriebenen Kriterien erfüllen und die Kohlenhydratkomponenten A und B ausmachen.

Neben diesen Kohlenhydratkomponenten A und B können noch andere Kohlenhydrate vorhanden sein. Dazu zählen 1.) die zwar löslichen, jedoch verdaubaren Kohlenhydrate, die gemäß der oben beschriebenen zweiten Stufe verdaubar sind, und 2.) die unlöslichen Kohlenhydrate, die resorbierbar/verdaubar oder auch nicht resorbierbar/verdaubar sind.

Diese unter 1.) und 2.) aufgezählten Kohlenhydrate können an sich in beliebigen Mengen neben den Kohlenhydratkomponenten A und B vorliegen je nach dem gewünschten Endprodukt. Vorzugsweise machen die unlöslichen Kohlenhydrate 0-10 Gew.-% der Kohlenhydratmischungen aus.

Die Kohlenhydratkomponente A kann beispielsweise aus einem oder mehreren der folgenden Kohlenhydraten bestehen: β-Galacto-Oligosaccharaide, α-Galacto-Oligosaccharide, Fructo-Oligosaccharide, Fuco-Oligosaccharide, Manno-Oligosccharide, Xylo-Oligosaccharide, Sialyl-Oligosaccharide, N-Glycoprotein-Oligosaccharide, O-Glycoprotein-Oligosaccharide, Glycolipid-Oligosaccharide, Cello-Oligosaccharide, Chitosan-Oligosaccharide, Chitin-Oligosaccharide, Galacturono-Oligosaccharide, Glucurono-Oligosaccharide, β-Glucan-Oligosaccharide, Arabinoxylo-Oligosaccharide, Arabinogalacto-Oligosaccharide, Xylogluco-Oligosaccharide, Galactomanno-Oligosaccharide, Rhamno-Oligosaccharide.

Die Kohlenhydratkomponente B kann beispielsweise aus einem oder mehreren der folgenden Kohlenhydraten bzw. Sacchariden aufgebaut sein:
Lösliche: Fructane, Galactane, Fucoidane, Arabinane, Xylane, Xanthane, β-Glucane, Galacturonane, N-Glycane, O-Glycane, Hyaluronsäuren, Chondroitine, Xyloglucane, Arabinogalactane, Alginate, Carageenane, Galactomannans, Arabinoxylane, Glycolipid-Glycane, Glycoptrotein-Glycane, Proteoglycane.

Durch die gezielte Kombination von Oligosacchariden und Polysacchariden und somit durch die gleichzeitige Anwesenheit der Kohlenhydratkomponente A und der Kohlenhydratkomponente B können die gesundheitsfördernden Mikroorganismen im Dickdarm wesentlich wirksamer gefördert werden als mit nur einer derartigen Kohlenhydratkomponente. So ist es durch die Verabreichung der erfindungsgemäßen Kombination möglich, eine normale Dickdarmflora sehr schnell wieder herzustellen, zu erhalten oder ein Abweichen der Darmflora in Belastungssituationen präventiv zu vermeiden und somit die bakterielle Besiedlung des Dickdarmes wirksamer zu beeinflussen als mit den bisher eingesetzten Kohlenhydraten.

Nach einer bevorzugten Ausführungsform bestehen sowohl die Kohlenhydratkomponente A als auch die Kohlenhydratkomponente B zu mindestens 80 Gew.-% aus Kohlenhydraten, die bifidogen sind und/oder Milchsäurebakterien fördern. Durch eine derartige Kombination von über diese Eigenschaften verfügenden Oligosacchariden und Polysacchariden kann das Wachstum der Milchsäurebakterien überraschenderweise wesentlich stärker gefördert werden als dies mit Oligosacchariden oder Polysacchariden alleine der Fall ist. Dabei werden nicht nur Milchsäurebakterien, die auf natürliche Weise im Darm vorhanden sind, sondern auch solche in ihrem Wachstum gefördert, das sogar selektiv sein kann, die exogen zugeführt werden.

Neben dieser indirekten Wirkung über die Bakterien selbst und deren Stoffwechselprodukte wie kurzkettige Fettsäuren (Butyrat, Propionat etc.) und damit pH-Effekte und Stimulation von Colonozyten werden auch direkte physikalische Effekte wie Peristaltik, Wassergehalt, Stuhlvolumen, mechanische Wirkung auf die Darmmukosa durch die erfindungsgemäßen Kohlenhydratmischungen positiv beeinflußt.

Die erfindungsgemäßen Kohlenhydratmischungen verfügen somit nicht nur über einen nutritiven Effekt sondern auch über ein breites Wirkungsspektrum. Mit den erfindungsgemäßen Mischungen können neben den oben aufgeführten biologischen Wirkungen auch noch folgende erzielt werden: Stabilisierung einer natürlichen Mikroflora, Verhinderung der Adhäsion von pathogenen Substanzen/Organismen wie Toxinen, Viren, Bakterien, Pilzen, transformierten Zellen und Parasiten, Auflösung von Komplexen von Toxinen, Viren, Bakterien, Pilzen und anderen Pathogenen mit körpereigenen Zellen sowie deren Ausschleusung aus dem Körper und Beschleunigung der Wundheilung.

Damit eignen sich die erfindungsgemäßen Mischungen zur Prophylaxe und/oder Behandlung von Symptomen/Erkrankungen, die im Zusammenhang mit einer gestörten Darmflora beispielsweise in Folge der Assoziation/Adhäsion der genannten Substanzen und Organismen an Epithelien oder andere körpereigene Zellen stehen.

Die Kohlenhydrate bzw. Saccharide der Kohlenhydratkomponenten A und B unterscheiden sich primär in ihrer Größe. Als besonders effektiv haben sich allerdings Mischungen herausgestellt, bei denen die Kohlenhydrate bzw. Saccharide der Kohlenhydratkomponente A einerseits und der Kohlenhydratkomponente B andererseits unterschiedlicher Struktur sind.-Diese unterschiedliche Struktur kann beispielsweise die Monosaccharid-Zusammensetzung betreffen, wenn beispielsweise einerseits Fructane und andererseits Galactane Anwendung finden. Diese unterschiedliche Struktur kann auch die glycosidische Bindung betreffen. Sowohl die Monomer-Komposition als auch die Glycosidbindung können einen Einfluß auf das chemische Verhalten (beispielsweise Löslichkeit) und auf das physiologische Verhalten (beispielsweise Verdaubarkeit) haben.

Der Kern der erfindungsgemäßen Mischungen ist somit unter anderem darin zu sehen, daß unterschiedlich große Kohlenhydrate zur Anwendung kommen, die vorzugsweise noch mindestens zwei unterschiedlichen "Klassen" angehören. Bei einer Verabreichung derartiger Mischungen kann ein synergistischer Effekt bezüglich der präbiotischen Wirkungen der einzelnen Substanzgruppen A und B auftreten.

Die Kohlenhydrate der Komponente A können dabei nicht nur einer Substanzklasse zugehörig sein, sondern auch aus mehreren aufgebaut sein (beispielsweise A: Galacto-Oligosaccharide plus Fuco-Oligosaccharide), während die Kohlenhydrate der Komponente B ebenfalls aus einer Substanzklasse und auch aus mehreren Substanzklassen stammen können (beispielsweise B:Inuline plus Xylane).

Erfindungsgemäß machen die Kohlenhydratkomponente A 90 Gew.-% und die Kohlenhydratkomponente B 10 Gew.-% der insgesamt vorhandenen Kohlenhydrate der Komponente A+B aus.

Erfindungsgemäße Mischungen sind solche, bei denen 80 bis 100 Gew.-% der Kohlenhydrate der Kohlenhydratkomponente A zur Gruppe der Galacto-Oligosaccharide und 80 bis 100 Gew.-% der Kohlenhydrate der Kohlenhydratkomponente B zur Gruppe der Fructo-Polysaccharide gehören. Galacto-Oligosaccharide setzen sich aus Galactoseresten in unterschiedlicher, besondere aber in β1-4 und β1-6 glycosidischer Bindung zusammen. Am reduzierenden Ende kann in β1-4 glycosidischer Bindung eine Glucose vorliegen. Fructo-Polysaccharide, zu denen die Fructane, Inuline und Levane gehören, setzen sich aus Fructoseresten in β2-1 und β-6 glycosidischer Bindung zusammen. Am reduzierenden Ende kann in β2-1 glycosidischer Bindung eine Glucose vorliegen.

Die Kohlenhydrate/Saccharide der Kohlenhydratkomponenten A und B weisen vorzugsweise keine Glucoseeinheiten in alpha 1-4 und/oder in alpha 1-6-Bindung auf.

Wenn im Rahmen der vorliegenden Unterlagen von Bereichen die Rede ist, dann sind mit der Bereichsangabe zumindest alle ganzzahligen Zwischenwerte und auch alle von dem weiteren Bereich umfaßte engere Bereiche umfaßt und offenbart. Das Gleiche gilt für die Angabe, daß mindestens 80 Gew.-% der Kohlenhydrate der Kohlenhydratkomponente A und mindestens 80 Gew.-% der Kohlenhydrate der Kohlenhydratkomponente B präbiotisch wirken bzw. Milchsäurebakterien fördern und/oder bifidogen sind. Der Begriff "mindestens 80 Gew.-%" bezeichnet somit zumindest alle Einzelwerte zwischen 80 Gew.-% und 100 Gew.-% und somit beispielsweise 81, 82, 83, 84, 85, 86, 87, 88, 89, 90, 91, 92, 93, 94, 95, 96, 97, 98, 99 und 100 Gew.-%. Die Kohlenhydratkomponenten A und B können somit auch ausschließlich aus derartigen Kohlenhydraten bestehen.

Das Molekulargewicht der Polysaccharide kann dabei bis zu einige MDa betragen und auf partikuläre Kohlenhydrate ausgedehnt werden. In der Komponente B werden jedoch Polysaccharidemoleküle mit bis zu maximal 100 Monosaccharidbausteinen eingesetzt.

Zur Herstellung der erfindungsgemäßen Kohlenhydratmischungen kann man bisher bekannte und insbesondere für die Herstellung von Nahrungen bzw. Nahrungsmittel eingesetzte Kohlenhydrate und Kohlenhydratmischungen einsetzen. Auch ist es möglich, bereits durch technische Modifikation veränderte Rohstoffe zur Anwendung zu bringen. Die Herstellung der erfindungsgemäßen Mischungen kann dabei durch einfaches Mischen der entsprechend ausgewählten Kohlenhydrate bzw. Oligosaccharide und Polysaccharide bzw. der Kohlenhydratmischungen hergestellt werden. Die Ausgangskomponenten müssen dabei derart miteinander vermischt werden, daß die erfindungsgemäßen Parameter bei den fertigen erfindungsgemäßen Mischungen eingehalten werden.

Als Rohstoffe können dabei Speicherkohlenhydrate (Fructane, Galacto-Oligosaccharide aus Leguminiosen, Fucoidan, alpha Glucane, Laminarin, Karragenan, Mannane, Glactomannane, Agar), Pflanzengummi, N-glycosidisch gebundene Kohlenhydrate der Glycoproteine, O-glycosidisch gebundene Kohlenhydrate der Glycoproteine, Glycane der Glycolipide, enzymatisch hergestellte Kohlenhydrate (Galacto-Oligosaccharide, Gluco-Oligosaccharide, Xylo-Oligosaccharide), bakterielle Kohlenhydrate (wie Xanthane), sowie Oligosaccharide (Galacto-Oligosaccharide, Gluco-Oligosaccharide (aus α1-2 und α1-3 Glucoseresten, Xylo-Oligosaccharide), als auch Gerüstkohlenhydrate wie Cellulosen, Hemizellulosen (Arabinane, Galactane), Pectine, Chitine eingesetzt werden. Die Substanzen sollten vorzugsweise food-grade sein (s. Complex Carbohydrates in foods, British Nutrition Foundation. Chapmann & Hall, London 1990). Auch ist es möglich eine enzymatische Modifikation der Rohstoffe mit Hydrolasen (beispielsweise Glycosidasen, Tranglycosidasen und Lipasen), Transferasen, Isomerasen (beispielsweise Aldolasen und Ketolasen) Oxidoreduktasen (beispielsweise Oxidasen) und Reduktasen (beispielsweise Glucosedehydrogenasen, Lyasen (beispielsweise Polysaccaridlyase) und Ligasen der Rohstoffe und Produkte durchzuführen. Ferner ist es möglich, eine technische Modifikation der Rohstoffe und Produkte vorzunehmen, nämlich durch Druck (beispielsweise Extrusion) Temperatur (beispielsweise Karamelisierung), organische Synthesen, organische Modifizierung (beispielsweise Carboxymethylierung und Peracetylierung) saure und/oder basische Hydrolyse und Fraktionierung (beispielsweise nach Größe und/oder physikochemischen Parametern wie Ladung und Hydrophobizität).

Die erfindungsgemäßen Kohlenhydratmischungen setzen sich dabei im wesentlichen aus den nachstehend aufgeführten Monosacchariden und den daraus aufgebauten Oligosacchariden sowie Polysacchariden zusammen: D-Glucose, D-Fructose, D-Galactose, D-Mannose, L-Fucose, D-N-Acetylglucosamin, D-N-Acetylgalactosamin, D-Xylose, L-Rhamnose, D-Arabinose, D-Allose, D-Talose, L-ldose, D-Ribose, sowie Monosaccharide mit Carboxylgruppen wie D-Galacturonsäure, D-Glucuronsäure, D-Mannuronsäure und/oder deren methylierte Formen, sowie N-Acetylneuraminsäure, N-Glycolylneuraminsäure und/oder deren O-acetylierte Formen.

Diese Monomere und die darauf aufgebauten höheren Einheiten können außerdem durch -OSO₃H- und/oder -OPO₃H-Gruppen modifiziert sein.

Gegenstand der Erfindung sind auch die erfindungsgemäßen Kohlenhydratmischungen enthaltende, diätetische und pharmazeutische Mittel und die Verwendung der oben beschriebenen Kohlenhydratmischungen zur Herstellung von diätetische und pharmazeutische Mittel zur Förderung der humanen Dickdarmflora. Dieser Begriff "Förderung" stellt einen Sammelbegriff für die oben aufgeführten biologischen Wirksamkeiten dar. Dazu zählt insbesondere die Förderung des Milchsäurebakterienwachstums.

Die erfindungsgemäßen Mischungen können in folgenden Produkten vorhanden sein:
Frühgeborenen-Nahrung, Reifgeborenen-Nahrung, Kinder-Nahrung, Humanmilch Fortifier, klinische Nahrung (im allgemeinen kann die erfindungsgemäße Mischung in diesen Nahrungen einen Teil oder komplett andere Komponenten ersetzen z.B. Lactose, Maltodextrin oder Stärke bzw. der Nahrung zugesetzt werden), Pharmazeutika, Diätetisches Supplement (als Sachet in Getränke).

Nachstehend sind verschiedene bevorzugte Ausführungsformen darstellende Kohlenhydratmischungen beschrieben. Die Angaben beziehen sich dabei auf Gew.-% sofern nichts anderes angegeben ist. In den Beispielen ist dabei aufgeführt, zu welchen Kohlenhydratkomponenten A oder B die eingesetzten Kohlenhydrate gehören. Die Kohlenhydratkomponente A wird dabei lediglich mit A und die Kohlenhydratkomponente B lediglich mit B bezeichnet.

### Beispiel 1

### Zusammensetzung

90 % A = Galacto-Oligosaccharide
Transgalacto-Oligosaccharide, z.B. Elixor® (Fa. Borculo, enzymatisch aus Lactose mittels β-Galactosidase)
10 % B = Inulin
Inulin, z.B. Raftiline® HP (Fa. Orafti Extraktion aus Zichorien, physikalische Abtrennung der niedermolekularen Oligosaccharide)

Zur Herstellung der Transgalacto-Oligosaccharide (Elixor®) wird Lactose mit β-Galactosidase behandelt. Dabei wird die Lactose katalytisch in Galacto-Oligosaccharide überführt, wobei eine Vielzahl in ihrer Kettenlänge variierender Galacto-Oligosaccharide gebildet werden. Primär werden dabei Disaccharide und Trisaccharide mit 3 bzw. 2 Galactoseeinheiten erhalten.

### Beispiel 2

### Zusammensetzung

80 % A = Transgalacto-Oligosaccharide
10 % A = Galacturonsäure-Oligosaccharide
10 % B = Inulin

## Patentansprüche

1. Kohlenhydratmischungen für diätetische Nahrungen und Pharmazeutika wobei
die Kohlenhydratmischungen zwei unterschiedliche, im wesentlichen lösliche Kohlenhydratkomponenten A und B, die im Magen-Darm-Trakt unverdaut bleiben und nicht resorbiert bis zum Dickdarm gelangen, enthalten oder daraus bestehen,
die Kohlenhydratkomponente A aus mindestens einem Monosaccharid oder aus mindestens einem Oligosaccharid mit 2 bis 6 Monosaccharideinheiten oder aus einer Mischung aus zwei oder mehreren dieser Saccharide aufgebaut ist,
die Kohlenhydratkomponente B aus einem Polysaccharid mit 7 oder mehr Monosaccharideinheiten oder aus einer Mischung aus zwei oder mehreren Polysacchariden aufgebaut ist,
die Kohlenhydratkomponente A = 9 Gew.-% und die Kohlenhydratkomponente B = 10 Gew.-% der Summe der Kohlenhydratkomponenten A + B (=100 Gew.-%) ausmachen, mindestens 80 Gew.-% der Kohlenhydrate/Saccharide der Kohlenhydratkomponente A und B präbiotisch wirken,
die Kohlenhydrate/Saccharide welche die Kohlenhydratkomponente A ausmachen, eine andere Struktur besitzen als die Kohlenhydrate/Saccharide welche die Kohlenhydratkomponente B ausmachen, wobei 80-10 Gew.-% der Kohlenhydrate/Saccharide der Kohlenhydratkomponente A zur Gruppe der Galactooligosaccharide und 80-10 Gew.-% der Kohlenhydrate/Saccharide der Kohlenhydratkomponente B zur Gruppe der Fructopolysaccharide gehören, und
die Kohlenhydrate/Saccharide der Kohlenhydratkomponente B aus maximal bis zu 100 Monosaccharideinheiten aufgebaut sind.

2. Kohlenhydratmischungen nach Anspruch 1, **dadurch gekennzeichnet,**
**daß** mindestens 80 Gew.-% der Kohlenhydrate/Saccharide der Kohlenhydratkomponenten A und B Milchsäurebakterien fördern und/oder bifidogen sind.

3. Kohlenhydratmischungen nach einem der vorhergehenden Ansprüche, **dadurch gekennzeichnet,**
**daß** die Kohlenhydrate der Kohlenhydratkomponenten A und B keine Glucoseeinheiten in α1-4 und/oder in α1-6-Bindung aufweisen.

4. Kohlenhydratmischungen nach einem der vorhergehenden Ansprüche, **dadurch gekennzeichnet,**
**daß** sie neben den Kohlenhydraten/Sacchariden der Kohlenhydratkomponenten A und B ein unlösliches Kohlenhydrat oder ein lösliches und verdaubares Kohlenhydrat oder eine Mischung aus einem oder mehreren dieser Kohlenhydrate enthalten.

5. Kohlenhydratmischungen nach einem der vorhergehenden Ansprüche zur Förderung der humanen Dickdarmflora und zur Förderung des Milchsäurebakterienwachstums.

6. Diätetisches oder pharmazeutisches Mittel enthaltend eine Kohlenhydratmischung nach einem der vorhergehenden Ansprüche.

7. Verwendung der Kohlenhydratmischungen nach einem der Ansprüche 1-5 zur Herstellung von Babynahrung.

## Claims

1. Carbohydrate mixtures for dietetic food products and pharmaceuticals wherein:
said carbohydrate mixtures contain or consist of two different, substantially soluble carbohydrate components A and B, which remain undigested in the gastrointestinal tract and enter the large intestine without being resorbed,
the carbohydrate component A is composed of at least one monosaccharide or of at least one oligosaccharide having 2 to 6 monosaccharide units or of a mixture of two or of more of these saccharides,
the carbohydrate component B is composed of one polysaccharide having 7 or more monosaccharide units or of a mixture of two or of more polysaccharides,
the carbohydrate component A = 90 weight percent and the carbohydrate component B = 10 weight percent of the sum of the carbohydrate components A + B (= 100 weight percent),
at least 80 weight percent of the carbohydrates / saccharides of the carbohydrate components A and B have a prebiotic effect,
the carbohydrates / saccharides which constitute the carbohydrate component A, have a different structure than the carbohydrates /saccharides which constitute the carbohydrate component B,
80 to 100 weight percent of the carbohydrates / saccharides of the carbohydrate component A belong to the galacto-oligosaccharide group and 80 to 100 weight percent of the carbohydrates / saccharides of the carbohydrate component B belong to the fructopolysaccharide group, and
the carbohydrates / saccharides of the carbohydrate component B are composed of a maximum of up to 100 monosaccharide units.

2. Carbohydrate mixtures according to claim 1, **characterized in that** at least 80 weight percent of the carbohydrates / saccharides of the carbohydrate components A and B promote lactic acid bacteria and/or are bifidogenic.

3. Carbohydrate mixtures according to any one of the preceding claims, **characterized in that** the carbohydrates / saccharides of the carbohydrate components A and B do not have any glucose units linked at the α 1-4 and/or α 1-6 position.

4. Carbohydrate mixtures according to any one of the preceding claims, **characterized in that**, apart from the carbohydrates /saccharides of the carbohydrate components A and B, they contain an insoluble carbohydrate or a soluble and digestible carbohydrate or a mixture of one or more of these carbohydrates.

5. Carbohydrate mixtures according to any one of the preceding claims, for promoting the flora of the large intestine in humans and for promoting the growth of lactic acid bacteria..

6. A dietetical or pharmaceutical composition containing a carbohydrate mixture according to any one of the preceding claims.

7. Use of carbohydrate mixtures according to any one of claims 1 to 5 for the production of infant formulas.

## Revendications

1. Mélanges d'hydrates de carbone pour aliments diététiques et produits pharmaceutiques, moyennant quoi les mélanges d'hydrates de carbone comprennent ou sont constitués de deux constituants d'hydrates de carbone différents essentiellement solubles A et B qui ne sont pas assimilés dans l'appareil digestif et qui arrivent non résorbés dans le gros intestin, le constituant d'hydrates de carbone A est constitué d'au moins un monosaccharide ou d'au moins un oligosaccharide avec entre 2 et 6 unités de monosaccharide ou d'un mélange de deux ou plusieurs de ces saccharides, le constituant d'hydrates de carbone B est constitué d'au moins un polysaccharide avec 7 ou plus unités de monosaccharide ou d'un mélange de deux ou plusieurs polysaccharides, le constituant d'hydrates de carbone A représente 90% en poids et le constituant d'hydrates de carbone B 10% en poids de la somme des constituants d'hydrates de carbone A + B (= 100% en poids), au moins 80% en poids des hydrates de carbone/saccharides des constituants d'hydrates de carbone A et B ont un effet prébiotique, les hydrates de carbone/saccharides qui constituent le constituant d'hydrates de carbone A ont une autre structure que les hydrates de carbone/saccharides qui constituent le constituant d'hydrates de carbone B, entre 80% et 100% en poids des hydrates de carbone/saccharides du constituant d'hydrates de carbone A font partie du groupe des galacto-oligosaccharides et entre 80% et 100% en poids des hydrates de carbone/saccharides du constituant d'hydrates de carbone B font partie du groupe des fructo-polysaccharides, et les hydrates de carbone/saccharides du constituant d'hydrates de carbone B sont constitués au maximum de 100 unités de monosaccharides.

2. Mélanges d'hydrates de carbone selon la revendication 1, **caractérisé en ce qu'**au moins 80% en poids des hydrates de carbone/saccharides des constituants d'hydrates de carbone A et B stimulent les bactéries de l'acide lactique et/ou sont bifidogènes.

3. Mélanges d'hydrates de carbone selon l'une des revendications précédentes, **caractérisé en ce que** les hydrates de carbone/saccharides des constituants d'hydrates de carbone A et B ne comportent aucune unité de glucose dans une liaison α1-4 et/ou dans une liaison α1-6.

4. Mélanges d'hydrates de carbone selon l'une des revendications précédentes, **caractérisé en ce que**, outre les hydrates de carbone/saccharides des constituants d'hydrates de carbone A et B, ils contiennent un hydrate de carbone insoluble ou un hydrate de carbone soluble et assimilable ou un mélange d'un ou plusieurs de ces hydrates de carbone.

5. Mélanges d'hydrates de carbone selon l'une des revendications précédentes pour la stimulation de la flore du gros intestin humain et pour la stimulation de la croissance des bactéries de l'acide lactique.

6. Produit diététique ou pharmaceutique contenant un mélange d'hydrates de carbone selon l'une des revendications précédentes.

7. Utilisation des mélanges d'hydrates de carbone selon l'une des revendications 1 à 5 pour la fabrication d'aliments pour bébés.
